(19) Europäisches Patentamt
European Patent Office
Office européen des brevets

(11) Veröffentlichungsnummer: **0 036 143**
**B1**

(12) # EUROPÄISCHE PATENTSCHRIFT

(45) Veröffentlichungstag der Patentschrift: **22.06.83**

(51) Int. Cl.³: **C 07 C143/66**, C 07 C139/00

(21) Anmeldenummer: **81101625.2**

(22) Anmeldetag: **06.03.81**

(54) **Verfahren zur Herstellung von 1-Amino-2-ethoxy-naphthalin-6-sulfonsäure.**

(30) Priorität: **18.03.80 DE 3010372**

(43) Veröffentlichungstag der Anmeldung: **23.09.81 Patentblatt 81/38**

(45) Bekanntmachung des Hinweises auf die Patenterteilung: **22.06.83 Patentblatt 83/25**

(84) Benannte Vertragsstaaten: **CH DE FR GB LI**

(56) Entgegenhaltungen:
**CH A 179 651**
**DD A 9 728**
**DE A 1 930 214**
**DE C 609 928**
**Fiat Final Report, No. 1313, pt1, Seite 269**

(73) Patentinhaber: **BAYER AG**
**Zentralbereich Patente, Marken und Lizenzen**
**D-5090 Leverkusen 1, Bayerwerk (DE)**

(72) Erfinder: **Pütter, Rolf, Dr.**
**Poststrasse 7**
**D-4000 Düsseldorf (DE)**
Erfinder: **Pfister, Theodor, Dr.**
**Sillerstrasse 51**
**D-5600 Wuppertal (DE)**
Erfinder: **Niese, Manfred, Dr.**
**Elisabeth-Langgaesser-Strasse 29**
**D-5090 Leverkusen (DE)**
Erfinder: **Wenzl, Peter, Dr.**
**Hahnenweg 4**
**D-5000 Köln 80 (DE)**

**EP 0 036 143 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Verfahren zur Herstellung von 1-Amino-2-ethoxy-naphthalin-6-sulfonsäure

Die Erfindung betrifft ein Verfahren zur Herstellung von 1-Amino-2-ethoxy-naphthalin-6-sulfonsäure aus 2-Hydroxy-naphthalin-6-sulfonsäure.

Es ist bekannt (DD-PS 9728), Ether der Amino-oxiverbindungen zweikerniger Kohlenwasserstoffe und ihrer kernsubstituierten Derivate, z. B. Sulfonsäuren, dadurch herzustellen, daß die Amino-oxiverbindungen bzw. ihre Derivate mit Säureanhydriden oder Säurechloriden in wäßriger Lösung in die N-Acylverbindungen überführt, letztere in bekannter Weise verethert werden und nachträglich die Acylgruppe durch Verseifung abgespalten wird.

Gemäß den Beispielen 2 und 3 der DD-PS 9728 erhält man 1-Amino-2-ethoxy-naphthalin-6-sulfonsäure, indem man in einer ersten Stufe aus 1-Amino-2-hydroxy-naphthalin-6-sulfonsäure durch Umsetzung mit Essigsäureanhydrid in verdünnter Essigsäure 1-Acetamino-2-hydroxy-naphthalin-6-sulfonsäure darstellt, dieses Zwischenprodukt in einer zweiten Stufe mit Diethylsulfat oder mit Ethylchlorid in wäßrig-alkoholischer Natronlauge zu 1-Acetamino-2-ethoxy-naphthalin-6-sulfonsäure verethert und schließlich dieses Intermediat ohne Zwischenisolierung durch Erhitzen mit wäßriger Natronlauge zu 1-Amino-2-ethoxy-naphthalin-6-sulfonsäure entacetyliert.

Die Ausbeute an 1-Amino-2-ethoxy-naphthalin-6-sulfonsäure beträgt nur ca. 67 %, bezogen auf eingesetzte 1-Amino-2-hydroxy-naphthalin-6-sulfonsäure. Daher ist das in der DD-PS 9728 beschriebene Verfahren für eine industrielle Großproduktion nicht geeignet.

Weiterhin ist aus FIAT Final Report, N° 1313, Pt. 1, Seite 269 bekannt, 1-Amino-2-hydroxy-naphthalin-6-sulfonsäure durch Umsetzung von 2-Hydroxy-naphthalin-6-sulfonsäure-Salz (« Schäffer-Salz ») mit Natriumnitrit, Essigsäure und Schwefelsäure und anschließende Reduktion des hierbei gebildeten 1-Nitroso-2-hydroxy-naphthalin-6-sulfonsäure mit Zink und Schwefelsäure herzustellen. 1-Amino-2-hydroxy-naphthalin-6-sulfonsäure wird dabei in 70 %-iger Ausbeute erhalten. Nachteilig bei diesem Verfahren ist die für ein technisches Verfahren unbefriedigende Ausbeute sowie der Einsatz von Zink statt Eisen für die Reduktion und die Verwendung von Essigsäure und Schwefelsäure anstelle der kostengünstigeren Salzsäure.

Es wurde nun ein Verfahren zur Herstellung von 1-Amino-2-ethoxy-naphthalin-6-sulfonsäure aus 2-Hydroxy-naphthalin-6-sulfonsäure gefunden, das dadurch gekennzeichnet ist, daß man

a) 2-Hydroxy-naphthalin-6-sulfonsäure und wenigstens die äquimolare Menge eines Alkalinitrits in wäßriger Lösung oder Suspension vorlegt und unter Zugabe von Salzsäure in einem pH-Bereich von 2 bis 5 bei Temperaturen von 0 bis 20 °C umsetzt,

b) das gegebenenfalls nach Aussalzen erhaltene Umsetzungsprodukt mit überschüssigem Eisen in Gegenwart einer mindestens äquivalenten Menge an Eisen-(II)-Ionen, bezogen auf das nach Schritt a) erhaltene Umsetzungsprodukt, und in Gegenwart von Mineralsäure bei Temperaturen von 50 bis 120 °C in wäßriger Suspension reduziert und anschließend bei Temperaturen von 80 bis 110 °C das Reaktionsgemisch mit wäßrigem Alkalihydroxid in Gegenwart von Eisenoxid sowie gegebenenfalls in Gegenwart einer reduzierend wirkenden Verbindung behandelt,

c) das nach Schritt b) erhaltene Produkt mit überschüssigem Acetanhydrid in wäßriger Lösung oder Suspension, gegebenenfalls nach Zugabe von Mineralsäure, in einem pH-Bereich von 3 bis 10 bei Temperaturen von 0 bis 100 °C umsetzt und

d) das gegebenenfalls nach Aussalzen erhaltene Produkt mit einem Ethylierungsmittel in Gegenwart von Säurebindemitteln in einem wäßrig-organischen Lösungs- oder Verdünnungsmittel, gegebenenfalls in Gegenwart von Kaliumverbindungen, im pH-Bereich von 8 bis 14 bei Temperaturen von 20 bis 150 °C umsetzt und anschließend das Umsetzungsprodukt mit wäßrigem Alkalihydroxid bei Rückflußtemperatur entacetyliert.

Das erfindungsgemäße Verfahren kann beispielsweise durch folgendes Formelschema dargestellt werden :

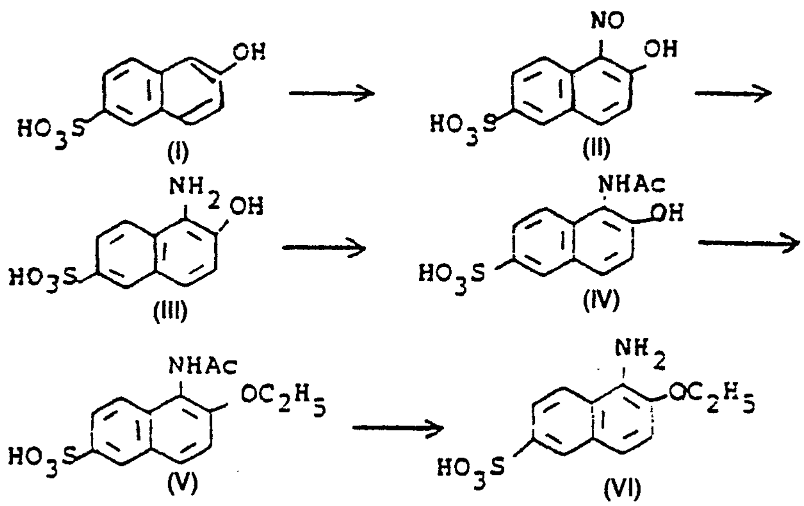

(I) (II) (III) (IV) (V) (VI)

Die Formeln (I) bis (VI) zeigen jeweils die freien Säuren. Sie stehen jedoch auch für deren Alkali- oder Erdalkalisalze, insbesondere für die Natrium- und Kaliumsalze, sowie für die Mischungen der Säuren mit ihren Salzen und die Mischungen der Salze untereinander.

Das erfindungsgemäße Verfahren wird im allgemeinen wie folgt durchgeführt:

Zu einer wäßrigen Lösung oder Suspension aus 2-Hydroxy-naphthalin-6-sulfonsäure (I) und einem Alkalinitrit, wie Lithium-, Natrium-, Kalium- und/oder Rubidiumnitrit, vorzugsweise Natriumnitrit, wird bei Temperaturen von 0 bis 20 °C, vorzugsweise 5 bis 15 °C, Salzsäure so zudosiert, daß sich der pH-Wert des Reaktionsgemisches in einem Bereich von 2 bis 5, vorzugsweise 3 bis 4, 5, einstellt.

Das Alkalinitrit wird nach dem erfindungsgemäßen Verfahren in wenigstens der äquimolaren Menge, bezogen auf die 2-Hydroxy-naphthalin-6-sulfonsäure, eingesetzt. Bevorzugt werden 1,00 bis 1,2 Mol Alkalinitrit, bezogen auf 1 Mol 2-Hydroxy-naphthalin-6-sulfonsäure, eingesetzt.

Die Salzsäure kann sowohl in wäßriger als auch in konzentrierter Form dem Reaktionsgemisch zugegeben werden. Beispielsweise kann die Salzsäure als 10 bis 40 gew.-%ige Salzsäure, bevorzugt als 25 bis 35 gew.-%ige Salzsäure, zugegeben werden.

Das gegebenenfalls nach mehrstündigem Rühren (etwa 1 bis 5 Stunden) oder nach Stehenlassen über Nacht durch Zugabe von beispielsweise Natriumchlorid ausgesalzene Nitrosierungsprodukt (II) wird dann mit überschüssigem Eisen, bevorzugt 10 bis 30 Mol Eisen, bezogen auf 1 Mol Nitrosierungsprodukt, in Gegenwart einer mindestens äquivalenten Menge an Eisen(II)-Ionen, bezogen auf das Nitrosierungsprodukt, und in Gegenwart von Mineralsäure, wie Salzsäure und/oder Schwefelsäure, vorzugsweise Salzsäure, in wäßriger Suspension bei Temperaturen von 50 bis 120 °C, vorzugsweise bei 80 bis 110 °C, umgesetzt.

Für das erfindungsgemäße Verfahren ist es von Bedeutung, daß bei Reaktionsbeginn eine der Menge an Nitrosierungsprodukt mindestens äquivalente Menge an Eisen(II)-Ionen in gelöster Form in der wäßrigen Suspension vorliegt.

Als Eisen(II)-Ionen können in das erfindungsgemäße Verfahren eingesetzt werden: Eisen(II)-Chlorid und/oder Eisen(II)-Sulfat, bevorzugt Eisen(II)-Chlorid. Bevorzugt werden 0,5 bis 1,5 Mol Eisen(II)-Ionen pro Mol Nitrosierungsprodukt vorgelegt.

Man geht dabei so vor, daß man eine entsprechende Menge eines Eisen(II)-Salzes in der wäßrigen Suspension löst, oder dieses Salz in situ durch entsprechenden Säureeinsatz erzeugt.

Nach der Reduktion wird das Reaktionsgemisch mit wäßrigem Alkalihydroxid, wie Lithium-, Natrium-, Kalium- und/oder Rubidiumhydroxid, bevorzugt Natriumhydroxid, in Gegenwart von Eisenoxid, wie Eisen(II), (III)-Oxid, und gegebenenfalls in Gegenwart einer reduzierend wirkenden Verbindung, wie Natriumsulfit, Natriumdithionit, Natrium-hydromethansulfinat (Rongalit ®), bevorzugt Natriumsulfit, bei Temperaturen von 80 bis 110 °C, bevorzugt 85 bis 100 °C, behandelt.

Die Menge an zuzusetzendem Eisenoxid ist nicht kritisch. Im allgemeinen gibt man 0,01 bis 0,5 Mol, bevorzugt 0,1 bis 0,3 Mol, Eisenoxid, bezogen auf eingesetzte 1-Nitroso-2-hydroxy-naphthalin-6-sulfonsäure(II), dem Reaktionsgemisch hinzu.

Auch die Menge an reduzierend wirkenden Verbindungen, die evtl. dem Reaktionsgemisch zugesetzt wird, kann in weiten Grenzen schwanken. Üblicherweise gibt man 0,005 bis 0,1, bevorzugt 0,01 bis 0,05 Mol, an reduzierend wirkender Substanz, bezogen auf eingesetztes Nitrosierungsprodukt (II), hinzu.

Die Salzsäure kann sowohl in wäßriger als auch in konzentrierter Form dem Reaktionsgemisch zugegeben werden. Zweckmäßigerweise wird die Salzsäure in konzentrierter Form zugesetzt. Die Menge der Salzsäure kann in weiten Bereichen schwanken. Im allgemeinen setzt man bei der Reduktion 0,1 bis 5,0 Mol, bevorzugt 0,5 bis 3,0 Mol, Salzsäure, bezogen auf 1 Mol 1-Nitroso-2-hydroxy-naphthalin-6-sulfonsäure, hinzu.

Die Menge an wäßrigem Alkalihydroxid ist ebenfalls nicht kritisch. Üblicherweise gibt man 1,0 bis 5,0, bevorzugt 1,5 bis 3,0 Mol, wäßriges Alkalihydroxid, bezogen auf 1 Mol 1-Nitroso-2-hydroxynaphthalin-6-sulfonsäure, dem Reaktionsgemisch zu.

Zur Acetylierung der erhaltenen 1-Amino-2-hydroxy-naphthalin-6-sulfonsäure (III) wird deren wäßrige Lösung oder Suspension, nachdem man die bei der Behandlung des Reduktionsgemisches mit Eisenoxid anfallenden Eisenverbindungen durch z. B. Filtration abgetrennt hat, gegebenenfalls durch Zugabe von Mineralsäure, wie Salzsäure, auf einen pH-Wert von 3 bis 10, vorzugsweise 5 bis 8, gebracht, und bei Temperaturen von 0 bis 100 °C, vorzugsweise bei 20 bis 60 °C, mit überschüssigem Acetanhydrid, vorzugsweise 1,05 bis 1,3 Mol, pro Mol Aminoverbindung (III), umgesetzt.

Nach einer bevorzugten Ausführungsform wird das zur Reduktion benötigte Eisen zusammen mit den Eisen(II)-salzen in Wasser vorgelegt, diese Suspension auf die oben angegebene Reaktionstemperatur (50 bis 120 °C) aufgeheizt und währenddessen konzentrierte Salzsäure zugetropft. Dann wird das Nitrosierungsprodukt portionsweise in das Eisen/Eisen(II)-Salz/Wasser-Gemisch eingetragen. Zum Reaktionsgemisch werden anschließend Natriumsulfit und Eisenoxid gegeben und Stickstoff übergeleitet. Anschließend behandelt man das Reaktionsgemisch mit Natronlauge bei 80 bis 100 °C und filtriert die ausfallenden Eisenverbindungen ab.

Die Acetylierung der 1-Amino-2-hydroxy-naphthalin-6-sulfonsäure (III) erfolgt dann in der zuvor beschriebenen Weise.

Die nach der Acetylierung, gegebenenfalls nach Aussalzen mit z. B. Natriumchlorid, erhaltene 1-Acetamino-2-hydroxy-naphthalin-6-sulfonsäure (IV) wird anschließend mit einem Ethylierungsmittel in

Gegenwart von Säurebindemitteln in einem wäßrig-organischen Lösungs- oder Verdünnungsmittel im pH-Bereich von 8 bis 14 bei Temperaturen von 20 bis 150 °C umgesetzt. Unter Umständen kann es zur Aufrechterhaltung des pH-Bereiches erforderlich werden, während der Reaktion noch Säurebindemittel hinzuzugeben.

Als Ethylierungsmittel können beispielsweise verwendet werden : Ethylchlorid, Ethylbromid, Ethyljodid, Diethylsulfat oder Triethylphosphit, bevorzugt Ethylchlorid oder Diethylsulfat.

Die Ethylierungsmittel werden im allgemeinen in Mengen von 1 bis 5 Mol, bevorzugt 1,5 bis 3 Mol, bezogen auf 1 Mol 1-Acetamino-2-hydroxy-naphthalin-6-sulfonsäure, eingesetzt.

Als Säurebindemittel können die üblicherweise in wäßriger Lösung verwendeten Basen eingesetzt werden. Als solche kommen insbesondere Alkalihydroxide, wie Natrium- und/oder Kaliumhydroxid, sowie Alkalicarbonate, wie Natrium- und/oder Kaliumcarbonat, in Betracht.

Die Säurebindemittel können in Mengen von 1 bis 5 Mol, bevorzugt von 1,2 bis 3 Mol, bezogen auf 1 Mol 1-Acetamino-2-hydroxy-naphthalin-6-sulfonsäure, eingesetzt werden.

Als wäßrig-organische Lösungs- oder Verdünnungsmittel kommen Gemische aus Wasser und mit Wasser mischbaren organischen Lösungs- oder Verdünnungsmitteln in Frage. Hierzu gehören insbesondere Alkohole, wie Methanol, Ethanol, n- und iso-Propanol, Ketone, wie Aceton und Methylethylketon, sowie cyclische Ether, wie Tetrahydrofuran und Dioxan.

Die Menge an zugesetzten wäßrig-organischen Lösungs- oder Verdünnungsmitteln beträgt im allgemeinen 0,3 bis 3 : 1, bevorzugt 0,5 bis 2 : 1 Mol, bezogen auf 1 Mol 1-Acetamino-2-hydroxy-naphthalin-6-sulfonsäure.

Nach einer bevorzugten Variante des erfindungsgemäßen Verfahrens wird als Veretherungs- bzw. Ethylierungsmittel Diethylsulfat eingesetzt. Man führt dann die Veretherungsreaktion so durch, daß 1-Acetamino-2-hydroxy-naphthalin-6-sulfonsäure in einem Gemisch aus Wasser und einem der o. g. organischen Lösungs- oder Verdünnungsmittel (Mischungsverhältnis 1 : 1 bis 1 : 10, vorzugsweise 1 : 2 bis 1 : 8) vorgelegt und der pH-Wert durch Basenzugabe auf einen Wert von 8 bis 12, bevorzugt 9 bis 11, eingestellt wird. Es ist darauf zu achten, daß der pH-Wert während der Reaktion in dem angegebenen Bereich gehalten wird. Bei einer Temperatur von 30 bis 90 °C wird dann Diethylsulfat in einer Menge von 1 bis 3 Mol, vorzugsweise 1,5 bis 2,5 Mol, je Mol 1-Acetamino-2-hydroxy-naphthalin-6-sulfonsäure zugetropft.

Nach einer anderen bevorzugten Variante des erfindungsgemäßen Verfahrens wird Ethylchlorid als Veretherungsmittel eingesetzt. Diese Variante wird in einem Autoklaven, vorzugsweise in Gegenwart von Kaliumverbindungen, wie Kaliumhydroxid, Kaliumcarbonat, Kaliumchlorid und/oder Kaliumacetat, bevorzugt Kaliumhydroxid und/oder Kaliumcarbonat, unter Verwendung eines der o. a. organischen Lösungs- oder Verdünnungsmittel, vorzugsweise Methanol und/oder Ethanol, im Gemisch mit Wasser, durchgeführt. Dabei werden die Kaliumverbindungen in etwa der äquimolaren Menge, bezogen auf 1-Acetamino-2-hydroxy-naphthalin-6-sulfonsäure, zugesetzt. Vorzugsweise gibt man 0,9 bis 1,5 Mol Kaliumverbindung pro Mol 1-Acetamino-2-hydroxynaphthalin-6-sulfonsäure hinzu.

Das Ethylchlorid wird in einer Menge von 1 bis 5 Mol, vorzugsweise 1,5 bis 3 Mol, pro Mol 1-Acetamino-2-hydroxy-naphthalin-6-sulfonsäure eingesetzt.

Während der Umsetzung ist wieder darauf zu achten, daß der pH-Wert des Reaktionsgemisches bis zum Reaktionsende nicht unter 8 sinkt. Gegebenenfalls wird während der Reaktion weitere Alkalilauge zugesetzt.

Die Entacetylierung des veretherten Produkts kann anschließend nach Abdestillieren des organischen Lösungs- oder Verdünnungsmittels auf bekannte Weise, z. B. durch Erhitzen auf Rückflußtemperatur mit wäßrigem Alkalihydroxid, wie Natriumhydroxid und/oder Kaliumhydroxid, durchgeführt werden. Dabei wird im allgemeinen das wäßrige Alkalihydroxid im Überschuß eingesetzt.

Das nach der Entacetylierung anfallende kristalline Produkt kann durch Vakuumfiltration isoliert werden ; der Gehalt an 1-Amino-2-ethoxy-naphthalin-6-sulfonsäure (VI) wird z. B. durch Diazotierung bestimmt.

Es ist außerordentlich überraschend, daß nach dem erfindungsgemäßen Verfahren 1-Amino-2-ethoxy-naphthalin-6-sulfonsäure ausgehend von 2-Hydroxy-naphthalin-6-sulfonsäure in einer mehrstufigen Reaktion in besonders guten Ausbeuten und Reinheiten erhalten wird.

Dies ist deshalb so überraschend, da nach dem Verfahren der DD-PS 9728 Ausbeuten an 1-Amino-2-ethoxy-naphthalin-6-sulfonsäure erhalten werden, die unter 70 % liegen, obwohl dieses Verfahren bereits von 1-Amino-2-hydroxy-naphthalin-6-sulfonsäure ausgeht, also zwei Verfahrensschritte weniger beinhaltet.

Die erfindungsgemäß hergestellte 1-Amino-2-ethoxy-naphthalin-6-sulfonsäure kann zur Herstellung von Azofarbstoffen verwendet werden (vgl. US-PS 2 636 030).

Die nachfolgenden Beispiele sollen das erfindungsgemäße Verfahren verdeutlichen, ohne es jedoch auf diese Beispiele einzuschränken.

Beispiel 1

284 g (78,9 %ig = 224 g 100 %ig = 1,0 Mol) 2-Hydroxy-naphthalin-6-sulfonsäure und 69,0 g (1,0 Mol) Natriumnitrit werden in 2,6 l Wasser vorgelegt. Zu dieser Mischung wird bei einer Temperatur von 10 °C

unter Rühren innerhalb von 3 Stunden tropfenweise 160 ml Salzsäure (18 %ig) gegeben, so daß sich ein pH-Wert von 4 einstellt. Die erhaltene Suspension wird mit 500 g Natriumchlorid versetzt und 2 Stunden lang gerührt. Danach wird abgesaugt, mit Kochsalzlösung gewaschen und getrocknet.

Man erhält 455 g (51,7 %ig = 235 g 100 %ig = 93 % der Theorie) 1-Nitroso-2-hydroxy-naphthalin-6-sulfonsäure in Form gelber Kristalle.

Beispiel 2

1 250 Teile des Natriumsalzes der 2-Hydroxy-naphthalin-6-sulfonsäure (62,7 %ig = 784 Teile 100 %ig) werden in 6 800 Teilen Wasser auf 70 bis 75 °C aufgeheizt bis eine klare Lösung entsteht. Dann wird die Lösung auf 5 bis 10 °C abgekühlt. Zu der entstandenen feinkristallinen Suspension werden 1 065 Teile 25 %ige Natriumnitritlösung gegeben. Man läßt nun bei 10 °C 410 Teile 30 %ige Salzsäure zulaufen, wobei sich ein pH-Wert von etwa 4 einstellt, rührt eine Stunde nach und salzt durch langsame Zugabe von 1 400 Teilen Kochsalz aus. Anschließend wird noch 2 Stunden nachgerührt. Nach Filtrieren und Waschen mit einer Kochsalzlösung erhält man 2 800 Teile wasserfeuchte 1-Nitroso-2-hydroxy-naphthalin-6-sulfonsäure (29,6 %ig = 829 Teile, 100 %ig = 94 % der Theorie).

Beispiel 3

In 500 ml Wasser werden 203 g Natriumsalz der 2-Hydroxy-naphthalin-6-sulfonsäure (55,4 %ig = 112,5 g 100 %ig = 0,5 Mol) gegeben und dieses Gemisch mit 152 g 25 %iger Natriumnitritlösung (0,55 Mol) versetzt. Nach Abkühlen des Gemisches auf etwa 5 bis 10 °C werden innerhalb einer Stunde 59,5 g 30 %ige Salzsäure zugegeben, wobei ein pH-Wert im Bereich von 2 bis 5 erhalten wird. Es wird eine Stunde nachgerührt, dann 100 g Kochsalz zugegeben und nach einer weiteren Stunde abgesaugt. Der Feststoff wird mit 100 ml Kochsalzlösung gewaschen und im Vakuum getrocknet.

Man erhält 217 g 1-Nitroso-2-hydroxy-naphthalin-6-sulfonsäure mit einem Gehalt von 56 % (= 121,5 g 100 %ig = 96 % der Theorie).

Beispiel 4

In einem 2 l Becherglas werden 400 g Eisen und 500 ml Wasser unter Rühren auf 90 bis 95 °C aufgeheizt. Während des Aufheizens gibt man 32 ml konzentrierte Salzsäure (30 %ig = 11 g 100 %ig = 0,302 Mol) hinzu. In das Eisen/Wasser-Gemisch werden innerhalb von 1,5 bis 2 Stunden bei 90 bis 95 °C 240 g (30 %ig = 73 g 100 %ig = 0,287 5 Mol) 1-Nitroso-2-hydroxy-naphthalin-6-sulfonsäure, die nach Beispiel 2 hergestellt wurde, eingetragen. Die Zugabe erfolgt in kleinen Portionen. Jeweils nach erfolgter Zugabe nimmt das Reaktionsgemisch eine tiefgrüne Färbung an, die nach kurzer Zeit wieder verschwindet. Schon bald nach Beginn der Zugabe der Nitrosoverbindung fällt die hellgraue Komplexverbindung von 1-Amino-2-hydroxy-naphthalin-6-sulfonsäure aus. Die nächste Portion der Nitrosoverbindung wird dann zugegeben, wenn die Grünfärbung verschwunden ist. Nach Ende des Eintragens der Nitrosoverbindung wird der Becherglasinhalt in einen 2 l Dreihalskolben gegeben und über das Gemisch Stickstoff geleitet. Zum Reaktionsgemisch gibt man 5 g Natriumsulfit und 20 g 70 %iges Eisen-(II), (III)-oxid. Danach läßt man bei 95 bis 100 °C innerhalb von 2 bis 5 Minuten 35 ml 50 %ige Natronlauge (27 g 100 %ig = 0,676 Mol) zulaufen. Dadurch wird der Komplex zerlegt und das gebundene Eisen fällt als schwarzer Niederschlag aus. Es wird abgesaugt, wobei in der Vorlage 90 ml konzentrierter Salzsäure (31 g 100 %ig = 0,85 Mol) vorgelegt werden. Beim Absaugen fällt in der Vorlage sogleich 1-Amino-2-hydroxy-naphthalin-6-sulfonsäure in Form grober Kristalle aus.

Zur Herstellung von 1-Acetamino-2-hydroxy-naphthalin-6-sulfonsäure wird das Gemisch aus 1-Amino-2-hydroxy-naphthalin-6-sulfonsäure und der Mutterlauge durch Zugabe von 28 ml 50 %iger Natronlauge (21 g 100 %ig = 0,54 mol) auf pH 8 eingestellt. Zu diesem Gemisch werden 0,5 g Natriumdithionit hinzugegeben. Dann läßt man 32 ml (34 g = 0,333 Mol) Essigsäureanhydrid bei einer Temperatur von 40 °C innerhalb von 5 Minuten zulaufen, rührt 5 Minuten nach und salzt das Produkt durch Zugabe von 240 g Natriumchlorid aus. Nach mehrstündigem Rühren bei 20 °C wird abgesaugt, mit gesättigter Kochsalzlösung gewaschen und getrocknet. Man erhält 110 g 1-Acetamino-2-hydroxy-naphthalin-6-sulfonsäure in Form fast farbloser Kristalle (60,4 %ig = 66,3 g, 100 %ig = 0,236 Mol).

Die entspricht einer Ausbeute von 82 % der Theorie, bezogen auf 1-Nitroso-2-hydroxynaphthalin-6-sulfonsäure, bzw. 77 % der Theorie, bezogen auf 2-Hydroxynaphthalin-6-sulfonsäure.

Beispiel 5

In einem 2 l Becherglas werden 400 g Eisen, 500 ml Wasser, 42 g Eisen-(II)-sulfat · 7 aq (0,151 Mol) oder 27 g Eisen-(III)-chlorid · 6 aq (0,1 Mol) sowie 3 ml konzentrierter Salzsäure miteinander gemischt und die Mischung auf 95 bis 100 °C erhitzt.

Zu diesem Gemisch werden innerhalb von 1,5 bis 2 Stunden 240 g (30 %ig = 73 g 100 %ig = 0,287 5 Mol) 1-Nitroso-2-hydroxy-naphthalin-6-sulfonsäure, die nach Beispiel 2 hergestellt wurde, eingetragen.

Das Reaktionsgemisch wird entsprechend dem Beispiel 4 weiterbehandelt.

Man erhält 110 g 1-Acetamino-2-hydroxy-naphthalin-6-sulfonsäure (60,4 %ig = 66,3 g 100 %ig = 0,236 Mol). Dies entspricht einer Ausbeute von 82 % der Theorie, bezogen auf 1-Nitroso-2-hydroxynaphthalin-6-sulfonsäure bzw. 77 % der Theorie, bezogen auf 2-Hydroxynaphthalin-6-sulfonsäure.

Beispiel 6

60,8 g (92,6 %ig = 56,3 g 100 %ig = 0,2 Mol) gereinigte (umkristallisierte) 1-Acetamino-2-hydroxy-naphthalin-6-sulfonsäure, die nach Beispiel 4 hergestellt wurde, werden in einer Mischung aus 240 ml Ethanol und 40 ml Wasser suspendiert. Durch Zutropfen von 2 n-Natronlauge wird der pH-Wert auf 9,5 eingestellt. Bei einer Temperatur von 40 bis 50 °C werden innerhalb von 20 Minuten 46 ml (0,36 Mol) Diethylsulfat zugetropft, wobei der pH-Wert durch Zugabe von Natronlauge bei 9,5 bis 10,5 gehalten wird. Das Reaktionsgemisch wird noch 3 Stunden unter Halten des pH-Wertes bei der oben angegebenen Temperatur gerührt. Dann destilliert man den Alkohol ab und erhitzt das Gemisch nach Zugabe von 44 ml 50 %iger Natronlauge 2 Stunden lang unter Rückfluß. Nach Abkühlen des Gemisches wird das ausgefallene Produkt abgesaugt, mit gesättigter Kochsalzlösung gewaschen und im Vakuum getrocknet.

Man erhält 61,7 g (72,0 %ig = 44,4 g 100 %ig = 83 % der Theorie) 1-Amino-2-ethoxy-naphthalin-6-sulfonsäure in Form weißgrauer Kristalle.

Beispiel 7

60,8 g (92,6 %ig = 56,3 g 100 %ig = 0,2 Mol) gereinigte (umkristallisierte) 1-Acetamino-2-hydroxy-naphthalin-6-sulfonsäure, hergestellt nach Beispiel 4, werden analog Beispiel 6 verethert, jedoch mit der Abweichung, daß der pH-Wert im Bereich von 9,0 bis 10,0 erhalten wird und die Reaktionstemperatur ungefähr 80 °C (Rückflußtemperatur des Lösungsmittels) beträgt. Die Nachrührzeit verkürzt sich damit auf ca. 30 Minuten. Die Entacetylierung wird wie im Beispiel 6 beschrieben, durchgeführt.

Ausbeute : 50,8 g (78,6 %ig = 45,6 g 100 %ig = 85 % der Theorie) 1-Amino-2-ethoxy-naphthalin-6-sulfonsäure.

Beispiel 8

15,3 g (91,8 %ig = 14,0 g 100 %ig = 0,05 Mol) gereinigte (umkristallisierte) 1-Acetamino-2-hydroxy-naphthalin-6-sulfonsäure, hergestellt nach Beispiel 4, werden in 67 ml Methanol auf 45 °C erwärmt. Nach Einstellen eines pH-Wertes von 10 mit 2 n-Natronlauge werden bei einer Temperatur von 40 bis 50 °C 13,7 g (0,09 Mol) Diethylsulfat innerhalb von 30 Minuten zugetropft. Das Gemisch wird noch 2 Stunden bei 45 °C nachgerührt, wobei der pH-Wert von 10 gehalten wird. Die Entacetylierung wird wie im Beispiel 6 beschrieben, durchgeführt.

Ausbeute : 13,1 g (83,2 %ig = 10,9 g 100 %ig = 81,5 % der Theorie) 1-Amino-2-ethoxy-naphthalin-6-sulfonsäure.

Beispiel 9

15,3 g (91,8 %ig = 14,0 g 100 %ig = 0,05 Mol) gereinigte (umkristallisierte) 1-Acetamino-2-hydroxy-naphthalin-6-sulfonsäure, hergestellt nach Beispiel 4, werden in einer Mischung aus 60 ml Aceton und 10 ml Wasser auf 45 °C erwärmt. Der pH-Wert wird mit Natronlauge auf 9 eingestellt. Innerhalb von 10 Minuten werden dann 13,7 g (0,09 Mol) Diethylsulfat zugetropft. Der pH-Wert wird noch 4 Stunden durch Zugabe von Natronlauge bei 9,5 gehalten. Die Entacetylierung wird wie in Beispiel 6 beschrieben, durchgeführt.

Ausbeute : 12,9 g (83,6 %ig = 10,8 g 100 %ig = 81 % der Theorie) 1-Amino-2-ethoxy-naphthalin-6-sulfonsäure.

Beispiel 10

15,3 g (91,4 %ig = 14,0 g 100 %ig = 0,05 Mol) 1-Acetamino-2-hydroxy-naphthalin-6-sulfonsäure, hergestellt nach Beispiel 4, werden in einem Gemisch aus 67 ml Alkohol und 12 ml Wasser auf 45 °C erwärmt. Der pH-Wert wird mit 2 n-Natronlauge auf 10 eingestellt. 13,7 g (0,09 Mol) Diethylsulfat werden dann innerhalb von 20 Minuten zugetropft und der pH-Wert wird durch Zugabe von Natronlauge noch 4 Stunden bei 10 gehalten. Man destilliert den Alkohol ab und erhitzt nach Zugabe von 40 ml 18 %iger Salzsäure das Gemisch 2 Stunden lang unter Rückfluß zum Sieden. Die Aufarbeitung erfolgt wie in Beispiel 6 beschrieben.

Ausbeute : 16,2 g (70,8 %ig = 11,5 g 100 %ig = 85 % der Theorie) 1-Amino-2-ethoxy-naphthalin-6-sulfonsäure. Das Produkt ist nach dünnschichtchromatographischer Analyse mit ca. 3 % 1-Amino-2-hydroxy-naphthalin-6-sulfonsäure verunreinigt.

Beispiel 11

15,3 g (92,1 %ig = 14,0 g 100 %ig = 0,05 Mol) gereinigte (umkristallisierte) 1-Acetamino-2-hydroxy-naphthalin-6-sulfonsäure, hergestellt nach Beispiel 4, werden in einem 0,3 l Autoklaven mit 134 ml einer Lösung von 9,7 g (0,15 Mol) Ethylchlorid in Ethanol, 23 ml 2 n-Kalilauge, 3,5 g (0,025 Mol) Kaliumcarbonat und 2,6 g (0,025 Mol) Natriumcarbonat bei Raumtemperatur vermischt und dieses Gemisch wird 5 Stunden bei 120 °C intensiv gerührt. Der Autoklaveninhalt wird dann zur Trockne eingeengt und der Rückstand wird mit 37 ml Wasser und 10 ml 50 %iger Natronlauge 2 Stunden lang unter Rückfluß erhitzt. Nach Abkühlen wird abgesaugt, das Produkt mit gesättigter Kochsalzlösung gewaschen und bei 50 °C im Vakuum getrocknet. Man erhält 14,15 g (82 %ig = 11,6 g 100 %ig = 87 % der Theorie) 1-Amino-2-ethoxy-naphthalin-6-sulfonsäure.

Beispiel 12

6,42 kg (62 %ig = 4 kg 100 %ig) 1-Acetamino-2-hydroxy-naphthalin-6-sulfonsäure, hergestellt nach Beispiel 4, 0,98 kg Kaliumcarbonat und 0,75 kg Natriumcarbonat werden in einem 150 l Autoklaven vorgelegt. Nach Durchspülen des Autoklaven mit Stickstoff werden 22 kg (28 l) Ethanol zu dem Gemisch zugegeben. Der Autoklav wird verschlossen und unter gutem Rühren auf 120 °C aufgeheizt. Nun werden 7 kg (6,5 l) 2 n-Kalilauge und 2,75 kg (ca. 3 l) Ethylchlorid innerhalb von 1 bis 2 Stunden in den Autoklaven gepumpt. Das komplette Reaktionsgemisch wird noch 5 Stunden bei 120 °C gerührt. Nach Abkühlen des Reaktionsgemisches auf Raumtemperatur wird entspannt und zweimal der Autoklav mit Stickstoff durchgespült. Der Autoklaveninhalt wird dann in einen 250 ml V4A-Stahlkessel gegeben. Der Autoklav wird mit 5 kg 80 %igem Ethanol ausgespült und die Spüllösung wird ebenfalls in den Stahlkessel gegeben. Das sich in dem Stahlkessel befindende Gemisch wird zum Sieden erhitzt, wobei ca. 20 kg Alkohol abdestilliert werden. Man läßt dann langsam zu der siedenden Lösung 13 kg Wasser zulaufen und destilliert solange, bis kein Alkohol mehr übergeht. Nach Zugabe von 4,3 kg 50 %iger Natronlauge wird das Gemisch 4 Stunden lang bei 100 °C gerührt. Anschließend wird das Gemisch auf 20 °C abgekühlt, das ausfallende Produkt abgesaugt und mit 25 kg Kochsalzlösung gewaschen. Nach Trocknen erhält man 3,6 kg 1-Amino-2-ethoxy-naphthalin-6-sulfonsäure (Gehalt : 84,2 %ig entspricht 3,03 kg 100 %ig = 80 % der Theorie, bezogen auf 1-Acetamino-2-hydroxynaphthalin-6-sulfonsäure bzw. 61,6 % der Theorie, bezogen auf 2-Hydroxynaphthalin-6-sulfonsäure).

Beispiel 13

98,1 g 1-Acetamino-2-hydroxynaphthalin-6-sulfonsäure (71,7 %ig = 50,4 g 100 %ig = 0,25 Mol), hergestellt nach Beispiel 4, werden in einen 1,3 l Autoklaven gegeben. Dazu gibt man 34,6 g (0,25 mol) Kaliumcarbonat aufgeschlämmt in 500 ml Ethanol. Nach Aufheizen des Gemisches auf 120 °C werden gleichzeitig 115 ml 2 n-Kalilauge und 54 ml (74 g = 0,75 Mol) Ethylchlorid zugepumpt. Man läßt das Reaktionsgemisch etwas abkühlen, saugt den Salzrückstand ab und destilliert dann nach Zugabe von 100 g Wasser 460 g Ethanol (wasserhaltig) ab. Bei einer Temperatur von 98 °C werden zu dem Gemisch 50 ml 50 %ige Natronlauge zugetropft. Nach 2 Stunden wird das Reaktionsgemisch auf Raumtemperatur abgekühlt, das ausgefallene Produkt abgesaugt und mit 100 ml gesättigter Kochsalzlösung gewaschen. Nach Absaugen und Trocknen erhält man 76,1 g 1-Amino-2-ethoxy-naphthalin-6-sulfonsäure (78,3 %ig = 60,0 g 100 %ig = 90 % der Theorie, bezogen auf 1-Acetamino-2-hydroxynaphthalin-6-sulfonsäure bzw. 69,4 % der Theorie, bezogen auf 2-Hydroxynaphthalin-6-sulfonsäure).

Beispiel 14

Die Umsetzung wird analog Beispiel 13 durchgeführt, wobei aber anstelle von Ethanol die gleiche Menge Methanol eingesetzt wird. Man erhält 87,5 g 1-Amino-2-ethoxy-naphthalin-6-sulfonsäure (70,6 %ig = 61,8 g 100 %ig) = 92,5 % der Theorie, bezogen auf 1-Acetamino-2-hydroxynaphthalin-6-sulfonsäure bzw. 71,2 % der Theorie, bezogen auf 2-Hydroxynaphthalin-6-sulfonsäure).

Beispiel 15

12,5 Teile 1-Acetamino-2-hydroxy-naphthalin-6-sulfonsäure (67,5 %ig = 8,5 Teile 100 %ig), hergestellt nach Beispiel 4, und 4,2 Teile Kaliumcarbonat werden in einem Autoklaven in einem Gemisch aus 47,4 Gew.-Teilen Methanol und 9,0 Gew.-Teilen Wasser suspendiert. Nach Aufheizen des Autoklaven werden bei 120 °C gleichzeitig 6,2 Teile 25 %ige Kalilauge und 5,8 Teile Ethylchlorid zudosiert. Das Reaktionsgemisch wird analog Beispiel 12 aufgearbeitet. Erhalten werden 87 % der Theorie an 1-Amino-2-ethoxy-naphthalin-6-sulfonsäure, bezogen auf 1-Acetamino-2-hydroxynaphthalin-6-sulfonsäure bzw. 67,8 % der Theorie, bezogen auf 2-Hydroxynaphthalin-6-sulfonsäure.

Beispiel 16

25 Gew.-Teile 1-Acetamino-2-hydroxy-naphthalin-6-sulfonsäure (67,5 %ig = 17 Teile 100 %ig), hergestellt nach Beispiel 4, und 8,3 Teile Kaliumcarbonat werden in einem Autoklaven in einem Gemisch aus 48 Gew.-Teilen Methanol und 18 Gew.-Teilen Wasser suspendiert. Bei 120 °C werden 12,4 Teile

Kalilauge (25 %ig) und 11,6 Teile Ethylchlorid zugesetzt. Nach Ende der Umsetzung wird die erhaltene Lösung entsprechend Beispiel 12 aufgearbeitet. Man erhält nach dem Trocknen 23,6 Gew.-Teile 1-Amino-2-ethoxy-naphthalin-6-sulfonsäure (55 %ig = 12,98 Teile 100 %ig = 81 % der Theorie, bezogen auf 1-Acetamino-2-hydroxynaphthalin-6-sulfonsäure bzw. 62,4 % der Theorie, bezogen auf 2-Hydroxy-naphthalin-6-sulfonsäure).

**Ansprüche**

1. Verfahren zur Herstellung von 1-Amino-2-ethoxy-naphthalin-6-sulfonsäure aus 2-Hydroxy-naphthalin-6-sulfonsäure, dadurch gekennzeichnet, daß man

a) 2-Hydroxy-naphthalin-6-sulfonsäure und wenigstens die äquimolare Menge eines Alkalinitrits in wäßriger Lösung oder Suspension vorlegt und unter Zugabe von Salzsäure in einem pH-Bereich von 2 bis 5 bei Temperaturen von 0 bis 20 °C umsetzt,

b) das gegebenenfalls nach Aussalzen erhaltene Umsetzungsprodukt mit überschüssigem Eisen in Gegenwart einer mindestens äquivalenten Menge an Eisen(II)-ionen, bezogen auf das nach Schritt a) erhaltene Umsetzungsprodukt, und in Gegenwart von Mineralsäure bei Temperaturen von 50 bis 120 °C in wäßriger Suspension reduziert und anschließend bei Temperaturen von 80 bis 110 °C das Reaktionsgemisch mit wäßrigem Alkalihydroxid in Gegenwart von Eisenoxid sowie gegebenenfalls in Gegenwart einer reduzierend wirkenden Verbindung behandelt,

c) das nach Schritt b) erhaltene Produkt mit überschüssigem Acetanhydrid in wäßriger Lösung oder Suspension, gegebenenfalls nach Zugabe von Mineralsäure, in einem pH-Bereich von 3 bis 10 bei Temperaturen von 0 bis 100 °C umsetzt
und

d) das gegebenenfalls nach Aussalzen erhaltene Produkt mit einem Ethylierungsmittel in Gegenwart von Säurebindemitteln in einem wäßrig-organischen Lösungs- oder Verdünnungsmittel, gegebenenfalls in Gegenwart von Kaliumverbindungen, im pH-Bereich von 8 bis 14 bei Temperaturen von 20 bis 150 °C umsetzt und anschließend das Umsetzungsprodukt mit wäßrigem Alkalihydroxid bei Rückflußtemperaturen entacetyliert.

2. Verfahren nach Anspruch 1, dadurch gekennzeichnet, daß man 1,00 bis 1,2 Mol Alkalinitrit pro Mol 2-Hydroxy-naphthalin-6-sulfonsäure einsetzt.

3. Verfahren nach Ansprüchen 1 und 2, dadurch gekennzeichnet, daß man als Alkalinitrit Lithiumnitrit, Natriumnitrit, Kaliumnitrit und/oder Rubidiumnitrit einsetzt.

4. Verfahren nach Ansprüchen 1 bis 3, dadurch gekennzeichnet, daß man 10 bis 30 Mol Eisen, bezogen auf 1 Mol 1-Nitroso-2-hydroxy-naphthalin-6-sulfonsäure, einsetzt.

5. Verfahren nach Ansprüchen 1 bis 4, dadurch gekennzeichnet, daß man 0,5 bis 1,5 Mol Eisen(II)-ionen pro Mol 1-Nitroso-2-hydroxy-naphthalin-6-sulfonsäure einsetzt.

6. Verfahren nach Anspruch 5, dadurch gekennzeichnet, daß man als Eisen(II)-ionen, Eisen(II)-chlorid und/oder Eisen(II)-sulfat einsetzt.

7. Verfahren nach Ansprüchen 1 bis 6, dadurch gekennzeichnet, daß man nach der Reduktion das Reaktionsgemisch mit Lithiumhydroxid, Natriumhydroxid, Kaliumhydroxid und/oder Rubidiumhydroxid in Gegenwart von Eisen(II) (III)-oxid, gegebenenfalls in Gegenwart von Natriumsulfit, Natriumdithionit oder Natrium-hydroxy-methansulfinat (Rongalit ®), bei Temperaturen von 85 bis 100 °C behandelt.

8. Verfahren nach Ansprüchen 1 bis 7, dadurch gekennzeichnet, daß man das nach Schritt b) erhaltene Produkt mit 1,05 bis 1,3 Mol Acetanhydrid pro Mol Aminoverbindung umsetzt.

9. Verfahren nach Ansprüchen 1 bis 8, dadurch gekennzeichnet, daß man das Ethylierungsmittel in Mengen von 1 bis 5 Mol, bezogen auf 1 Mol 1-Acetamino-2-hydroxy-naphthalin-6-sulfonsäure, einsetzt.

10. Verfahren nach Ansprüchen 1 bis 9, dadurch gekennzeichnet, daß man als Ethylierungsmittel Diethylsulfat oder Ethylchlorid einsetzt.

**Claims**

1. Process for the preparation of 1-amino-2-ethoxy-naphthalene-6-sulphonic acid from 2-hydroxynaphthalene-6-sulphonic acid, characterised in that

a) 2-hydroxy-naphthalene-6-sulphonic acid and at least the equimolar amount of an alkali metal nitrite are initially introduced into the reaction vessel in aqueous solution or suspension and are reacted, with the addition of hydrochloric acid, in a pH range from 2 to 5 and at temperatures from 0 to 20 °C,

b) the reaction product obtained, if necessary after salting out, is reduced in aqueous suspension with excess iron in the presence of at least an equivalent amount of iron-II ions, relative to the reaction product obtained according to step a) and in the presence of mineral acid at temperatures from 50 to 120 °C, and the reaction mixture is then treated with aqueous alkali metal hydroxide in the presence of iron oxide and, if appropriate, in the presence of a reducing compound at temperatures from 80 to 110 °C,

c) the product obtained according to step b) is reacted with excess acetic anhydride in aqueous solution or suspension, if appropriate after adding mineral acid, in a pH range from 3 to 10 and at temperatures from 0 to 100 °C, and

d) the product obtained, if necessary after salting out, is reacted with an ethylating agent in the

presence of acid-binding agents and in an aqueous-organic solvent or diluent, if appropriate in the presence of potassium compounds, in a pH range from 8 to 14 and at temperatures from 20 to 150 °C, and the reaction product is then deacetylated at reflux temperatures with aqueous alkali metal hydroxide.

2. Process according to Claim 1, characterised in that 1.00 to 1.2 mols of alkali metal nitrite are employed per mol of 2-hydroxy-naphthalene-6-sulphonic acid.

3. Process according to Claims 1 and 2, characterised in that the alkali metal nitrite used is lithium nitrite, sodium nitrite, potassium nitrite and/or rubidium nitrite.

4. Process according to Claims 1 to 3, characterised in that 10 to 30 mols of iron are employed per mol of 1-nitroso-2-hydroxy-naphthalene-6-sulphonic acid.

5. Process according to Claims 1 to 4, characterised in that 0.5 to 1.5 mols of iron-II ions are employed per mol of 1-nitroso-2-hydroxy-naphthalene-6-sulphonic acid.

6. Process according to Claim 5, characterised in that the iron-II ions employed are iron-II chloride and/or iron-II sulphate.

7. Process according to Claims 1 to 6, characterised in that following the reduction the reaction mixture is treated with lithium hydroxide, sodium hydroxide, potassium hydroxide and/or rubidium hydroxide in the presence of iron-II/III oxide, and if appropriate in the presence of sodium sulphite, sodium dithionite or sodium hydroxymethanesulphinate (Rongalite ®), at temperatures of 85 to 100 °C.

8. Process according to Claims 1 to 7, characterised in that the product obtained according to step b) is reacted with 1.05 to 1.3 mols of acetic anhydride per mol of amino compound.

9. Process according to Claims 1 to 8, characterised in that the ethylating agent is employed in amounts of 1 to 5 mols per mol of 1-acetamino-2-hydroxy-naphthalene-6-sulphonic acid.

10. Process according to Claims 1 to 9, characterised in that diethyl sulphate or ethyl chloride is employed as the ethylating agent.

**Revendications**

1. Procédé de production d'acide 1-amino-2-éthoxynaphtalène-6-sulfonique à partir d'acide 2-hydroxy-naphtalène-6-sulfonique, caractérisé en ce que

a) on introduit au préalable de l'acide 2-hydroxynaphtalène-6-sulfonique et au moins la quantité équimolaire d'un nitrite alcalin en solution ou en suspension aqueuse et on les fait réagir, avec addition d'acide chlorhydrique, dans une plage de pH de 2 à 5 à des températures de 0 à 20 °C,

b) le produit réactionnel obtenu éventuellement après relargage, avec du fer en excès, est réduit en suspension aqueuse en présence d'une quantité au moins équivalente d'ions fer (II), par rapport au produit réactionnel obtenu selon l'étape a), et en présence d'un acide minéral à des températures de 50 à 120 °C, puis le mélange réactionnel est traité à des températures de 80 à 110 °C avec un hydroxyde alcalin aqueux en présence d'oxyde de fer ainsi que, le cas échéant, en présence d'un composé à action réductrice,

c) le produit obtenu selon l'étape b) est amené à réagir avec de l'acétanhydride en excès en solution ou en suspension aqueuse, éventuellement après addition d'un acide minéral, dans une plage de pH de 3 à 10 à des températures de 0 à 100 °C et

d) le produit obtenu éventuellement après relargage est amené à réagir avec un agent d'éthylation en présence d'accepteurs d'acides dans un solvant ou diluant hydro-organique, éventuellement en présence de composés de potassium, dans la plage de 8 à 14 à des températures de 20 à 150 °C et le produit réactionnel est ensuite désacétylé avec un hydroxyde alcalin aqueux, aux températures de reflux.

2. Procédé suivant la revendication 1, caractérisé en ce qu'on utilise 1,00 à 1,2 mole de nitrite alcalin par mole d'acide 2-hydroxynaphtalène-6-sulfonique.

3. Procédé suivant les revendications 1 et 2, caractérisé en ce qu'on utilise comme nitrite alcalin le nitrite de lithium, le nitrite de sodium, le nitrite de potassium et/ou le nitrite de rubidium.

4. Procédé suivant les revendications 1 à 3, caractérisé en ce qu'on utilise 10 à 30 moles de fer par rapport à une mole d'acide 1-nitroso-2-hydroxynaphtalène-6-sulfonique.

5. Procédé suivant les revendications 1 à 4, caractérisé en ce qu'on utilise 0,5 à 1,5 mole d'ions fer (II) par mole d'acide 1-nitroso-2-hydroxynaphtalène-6-sulfonique.

6. Procédé suivant la revendication 5, caractérisé en ce qu'on utilise comme ions fer-(II) le chlorure de fer-(II) et/ou le sulfate de fer-(II).

7. Procédé suivant les revendications 1 à 6, caractérisé en ce que, après la réduction, on traite le mélange réactionnel avec de l'hydroxyde de lithium, de l'hydroxyde de sodium, de l'hydroxyde de potassium et/ou de l'hydroxyde de rubidium en présence d'oxyde de fer-(II) (III), éventuellement en présence de sulfite de sodium, de dithionite de sodium ou d'hydroxyméthanesulfinate de sodium (Rongalit®) à des températures de 85 à 100 °C.

8. Procédé suivant les revendications 1 à 7, caractérisé en ce qu'on fait réagir le produit obtenu selon l'étape b) avec 1,05 à 1,3 mole d'acétanhydride par mole de composé aminé.

9. Procédé suivant les revendications 1 à 8, caractérisé en ce qu'on utilise l'agent d'éthylation en quantités de 1 à 5 moles, par rapport à 1 mole d'acide 1-acétamino-2-hydroxynaphtalène-6-sulfonique.

10. Procédé suivant les revendications 1 à 9, caractérisé en ce qu'on utilise comme agent d'éthylation le sulfate de diéthyle ou le chlorure d'éthyle.